# EUROPEAN PATENT SPECIFICATION

(11) **EP 3 291 831 B1**
(45) Date of publication and mention of the grant of the patent: **30.12.2020**
(21) Application number: 16724315.3
(22) Date of filing: 03.05.2016
(51) Int. Cl.: A61P 13/10, A61P 35/00, A61K 39/00, A61K 39/04

(54) **RECOMBINANT MYCOBACTERIUM AS AN IMMUNOTHERAPEUTIC AGENT FOR THE TREATMENT OF CANCER**
REKOMBINANTES MYCOBAKTERIUM ALS IMMUNTHERAPEUTIKUM ZUR BEHANDLUNG VON KREBS
MYCOBACTÉRIE DE RECOMBINAISON EN TANT QU'AGENT IMMUNOTHÉRAPEUTIQUE POUR LE TRAITEMENT DU CANCER

(30) Priority: 04.05.2015 EP 15166206; 23.12.2015 US 201562387407 P
(43) Date of publication of application: 14.03.2018
(73) Proprietor: Vakzine Projekt Management GmbH, 30625 Hannover (DE)
(72) Inventor: GRODE, Leander, 38110 Braunschweig (DE)
(74) Representative: Weickmann & Weickmann PartmbB
(86) International application number: PCT/EP2016/059872
(87) International publication number: WO 2016/177717

(56) References cited:
- EP-A1- 1 649 869
- WO-A1-2004/094469
- WO-A1-2012/085101
- AU-A1- 2014 210 617
- RENTSCH C.A. ET AL.: "VPM1002- a recombinant BCG with favourable preclinical toxicity and immunogenicity forpotential improvement of BCG immunotherapy for non-muscle invasive bladder cancer", EUR UROL SUPPL, vol. 13, E521, 11 April 2014 (2014-04-11), - 15 April 2014 (2014-04-15), XP002746389,
- Anonymous: "VPM1002BC in Recurrent Non-muscle Invasive Bladder Cancer", ClinicalTrials.gov , 10 February 2015 (2015-02-10), XP002746390, Retrieved from the Internet: URL:https://clinicaltrials.gov/ct2/show/NC T02371447 [retrieved on 2015-10-12]
- GRODE LEANDER ET AL: "Safety and immunogenicity of the recombinant BCG vaccine VPM1002 in a phase 1 open-label randomized clinical trial", VACCINE, vol. 31, no. 9, 3 January 2013 (2013-01-03), pages 1340-1348, XP028970807, ELSEVIER LTD, GB ISSN: 0264-410X, DOI: 10.1016/J.VACCINE.2012.12.053
- KUPFERSCHMIDT KAI: "Taking a new shot at a TB vaccine.", SCIENCE, vol. 334, no. 6062, 16 December 2011 (2011-12-16), pages 1488-1490, XP002746391, (NEW YORK, N.Y.) ISSN: 1095-9203
- Swiss Group for Clinical Cancer Research: "VPM1002BC in Recurrent Non-muscle Invasive Bladder Cancer", , 19 February 2015 (2015-02-19), Retrieved from the Internet: URL:https://clinicaltrials.gov/ct2/history /NCT02371447?V_1=View#StudyPageTop [retrieved on 2018-12-04]
- KAUFMANN STEFAN HE ET AL: "The BCG replacement vaccine VPM1002: from drawing board to clinical trial", EXPERT REVIEW OF VACC, EXPERT REVIEWS LTD, GB, vol. 13, no. 5, 1 May 2014 (2014-05-01), pages 619-630, XP008177774, ISSN: 1744-8395, DOI: 10.1586/14760584.2014.905746 [retrieved on 2014-04-07]

## Description

The invention relates to a urease-deficient recombinant *Mycobacterium bovis* (*M. bovis*) Bacillus Calmette Guerin (BCG) cell for use as an immunotherapeutic agent in the treatment of cancer, particularly in the treatment of solid tumors. More particularly, the invention relates to the immunotherapy of bladder carcinoma.

Urothelial bladder carcinoma is the 5th most common cancer. In the United States, about 75.000 new cases are diagnosed each year 4.5% of all new cancers, and approximately 15.600 deaths are expected. In Germany, about 16.000 new cases are diagnosed each year. Because a recurrence of disease is likely in bladder carcinoma, patients must undergo surveillance for an extended period.

Most bladder carcinomas begin in transitional epithelial cells that make up the inner lining of the bladder. As these tumors grow, they can invade the surrounding connective tissue and muscle. In advanced disease, tumors spread beyond the bladder to nearby lymph nodes or pelvic organs or metastasize to more distant organs such as lung, liver and bone.

The overall 5-year survival rate for bladder carcinoma is 77%, and this rate has not changed significantly over the last 10 years. When considered by stage, the 5-year relative survival rates for patients with tumors restricted to the inner layer of the bladder are 96% and 69%, respectively. The rates drop to 34% for those with disease that has spread locally beyond the bladder and to 6% with distant metastases.

For patients with non-muscle invasive bladder carcinoma, treatment usually involves a surgical removal of the tumor followed by chemotherapy, usually mitomycin C, within the bladder (so-called intravesical chemotherapy). After recovering from surgery, patients with a lower risk of disease progression may undergo surveillance or additional intravesical chemotherapy. Patients with moderate-to-high-grade disease often receive intravesical immunotherapy with an attenuated live bacterium BCG. BCG was the first FDA-approved immunotherapy and helps reduce the risk of bladder carcinoma recurrence by stimulating an immune response that targets the bacteria as well as any bladder carcinoma cells. In some patients, however, BCG therapy has found to be less effective, particularly after repeated administration.

Standard treatment for patients with muscle-invasive bladder carcinoma includes cisplatin-based chemotherapy followed by surgical removal of the bladder or radiation therapy and concomitant chemotherapy. Recurrent bladder carcinoma may be treated with combination therapy regimens, including gemcitabine plus cisplatin or methotrexate, vinblastine, doxorubicin plus cisplatin.

In the treatment of bladder carcinoma, tumor recurrence is a major concern, even for patients with low-grade disease and requires extensive follow-up. Better treatments, such as novel immunotherapies, might reduce recurrence rates and improve the survival of patients with bladder carcinoma.

A recombinant BCG strain expressing a phagolysosomal escape domain is described in WO 99/10496. The phagolysosomal escape domain enables the strain to escape from the phagosome of infected host cells by perforating the membrane of the phagosome. In order to provide an acidic phagosomal pH for optimal phagolysosomal escape activity, a urease-deficient recombinant strain was developed. This strain (strain BCG Pasteur 1173P₃) is disclosed in WO 2004/094469.

WO 2012/085101, discloses that a recombinant BCG strain expressing membrane-perforating listeriolysin (Hly) of *Listeria monocytogenes* and devoid of urease C induces superior protection against aerogenic challenge with *Mycobacterium tuberculosis*

(*Mtb*) as compared to parental BCG in a preclinical model. Further, it is shown that both the recombinant and the parenteral strain induce marked Th1 immune responses, whilst only the recombinant BCG strain elicits are profound Th17 response in addition.

Grode et al. (Vaccine, 31(9), 1340-1348, 2013), Kupferschmidt (Science 334 (6062), 1488-1490, 2011) and Kaufmann et al. (Expert Rev Vaccines 13 (5), 619-630, 2014) disclose the clinical efficacy and safety of a genetically modified BCG vaccine called VPM1002 (*Mycobacterium bovis* BCG ΔureC::Hly⁺) as vaccine directed against tuberculosis.

An internet excerpt retrieved from URL:https.//clinicaltrials.gov/ct2/history/NCT02371447?V 1=View#studyPageTop (19.02.2015), entitled "VPM1002 BC in recurrent non-muscle invasive bladder cancer" relates to a protocol of a clinical trial assessing the safety and efficacy of intravesical instillation of VPM1002BC in patients with recurrent non-muscle invasive bladder cancer (NMIBC) after transurethral resection of the bladder and standard BCG therapy. The exact identity/structure of VPM1002BC is however not described.

In the present study, it was found that a recombinant urease-deficient and listeriolysin-expressing recombinant BCG strain induces a superior immune response compared to the parental BCG in an animal model.

A subject-matter of the present invention is a urease-deficient recombinant *M*. *bovis* BCG cell from strain Danish subtype Prague which comprises a recombinant nucleic acid molecule encoding a fusion polypeptide comprising:
(a) a domain capable of eliciting an immune response comprising the amino acid sequence from aa.41 to aa.51 in SEQ ID No.2, and
(b) a Listeria phagolysosomal escape domain encoded by a nucleic acid molecule selected from
   (i) a nucleotide sequence comprising nucleotides 211-1722 as shown in SEQ ID No.1,
   (ii) a nucleotide sequence which encodes the same amino acid sequence as the sequence from (i), and
   (iii) a nucleotide sequence hybridising under stringent conditions with the sequence from (i) or (ii)
   for use in a human subject as immunotherapeutic agent in the treatment of bladder carcinoma, wherein the treatment comprises local administration of the immunotherapeutic agent by vesicular instillation into the urinary bladder, particularly after surgery.

According to the present invention it was found that vesicular instillation of a recombinant BCG cell into the bladder of rats surprisingly results in an increased infiltration of urinary bladder tissue by lymphocytes, particularly CD4- and CD8-positive lymphocytes resulting in a high incidence of focal and/or multifocal lymphocytic infiltration. In contrast thereto, the urinary bladder tissue of animals treated with standard BCG showed CD4- and CD8-positive lymphocytes only as single cell infiltrate levels (diffuse infiltration). Further, administration of a recombinant BCG cell did not raise any safety issues.

Presently, a phase I/II clinical trial assessing safety and efficacy of intravesical instillation of recombinant BCG in human patients with recurrent non-muscle invasive bladder carcinoma after standard BCG therapy is conducted.

Thus, the present invention also relates to a urease-deficient recombinant *M. bovis* BCG cell from strain Danish subtype Prague as described above for use as an immunotherapeutic agent in the treatment of solid tumors in order to obtain focal and/or multifocal lymphocytic infiltration, e.g. with CD4 and CD8 T cells at the site of administration. The recombinant *M. bovis* BCG cell of the present invention is particularly suitable for use in human medicine.

The immunotherapeutic agent is a live recombinant *M. bovis* BCG cell which comprises a recombinant nucleic acid molecule encoding a fusion polypeptide comprising (a) a domain capable of eliciting an immune response and (b) a phagolysosomal escape domain.

The Mycobacterium cell is an attenuated recombinant *M. bovis* BCG cell from strain Danish subtype Prague (Brosch et al., Proc. Natl. Acad. Sci. USA, 104 (2007), 5396-5601). The Mycobacterium cell is recombinant urease-deficient. In an especially preferred embodiment the ureC sequence of the Mycobacterium cell is inactivated (ΔUrec), e.g. by constructing a suicide vector containing a ureC gene disrupted by a selection marker gene, e.g. the hygromycin gene, transforming the target cell with the vector and screening for selection marker-positive cells having a urease negative phenotype. In an even more preferred embodiment, the selection marker gene, i.e. the hygromycin gene, is subsequently inactivated. In this embodiment, the cell is a selection marker-free recombinant Mycobacterium cell. Most preferably, the cell is selection marker-free recombinant BCG strain Danish subtype Prague characterized as recombinant BCG ΔUrec::Hly+.

The domain capable of eliciting an immune response is derived from antigen Ag85B from *M. bovis* BCG and comprises the amino acid sequence from aa.41 to aa.51 in SEQ ID NO.2.

The recombinant nucleic acid molecule further comprises a phagolysosomal escape domain, i.e. a polypeptide domain which provides for an escape of the fusion polypeptide from the phagolysosome into the cytosol of mammalian cells. The phagolysosomal escape domain is a Listeria phagolysosomal escape domain, which is described in US 5,733,151, wherein the phagolysosomal escape domain is derived from the listeriolysin gene (Hly) of L. monocytogenes and encoded by a nucleic acid molecule selected from: (a) a nucleotide sequence comprising nucleotides 211 - 1722 as shown in SEQ ID No.1, (b) a nucleotide sequence which encodes the same amino acid sequence as the sequence from (a), and (c) a nucleotide sequence hybridizing under stringent conditions with the sequence from (a) or (b).

Apart from the nucleotide sequence depicted in SEQ ID No.1 the present invention also comprises nucleic acid sequences hybridizing therewith. In the present invention the term "hybridization" is used as defined in Sambrook et al. (Molecular Cloning. A laboratory manual, Cold Spring Harbor Laboratory Press (1989), 1.101-1.104). In accordance with the present invention the term "hybridization" is used if a positive hybridization signal can still be observed after washing for one hour with 1 X SSC and 0.1 % SDS at 55°C, preferably at 62° C and more preferably at 68°C, particularly for 1 hour in 0.2 X SSC and 0.1 % SDS at 55°C, preferably at 62°C and more preferably at 68°C. A sequence hybridizing with a nucleotide sequence as per SEQ ID No.1 under such washing conditions is a phagolysosomal escape domain encoding nucleotide sequence preferred by the subject invention.

A nucleotide sequence encoding a phagolysosomal escape domain as described above may be directly obtained from a Listeria organism or from any recombinant source e.g. a recombinant *E.coli* cell containing the corresponding Listeria nucleic acid molecule or a variant thereof as described above.

Preferably, the recombinant nucleic acid molecule encoding for a fusion polypeptide contains a signal peptide encoding sequence. More preferably, the signal sequence is a signal sequence active in Mycobacteria, preferably in *M.bovis,* e.g. a native *M.bovis* signal sequence. A preferred example of a suitable signal sequence is the nucleotide sequence coding for the Ag85B signal peptide which is depicted in SEQ ID No.1 from nucleotide 1 to 120.

Further, it is preferred that a peptide linker be provided between the immunogenic domain and the phagolysosomal escape domain. Preferably, said peptide linker has a length of from 5 to 50 amino acids. More preferably, a sequence encoding a linker as shown in SEQ ID No.1 from nucleotide 154 to 210 or a sequence corresponding thereto as regards the degeneration of the genetic code.

The nucleic acid may be located on a recombinant vector. Preferably, the recombinant vector is a prokaryotic vector, i.e. a vector containing elements for replication or/and genomic integration in prokaryotic cells. Preferably, the recombinant vector carries the nucleic acid molecule of the present invention operatively linked with an expression control sequence. The expression control sequence is preferably an expression control sequence active in Mycobacteria, particularly in *M.bovis.* The vector can be an extrachromosomal vector or a vector suitable for integration into the chromosome. Examples of such vectors are known to the man skilled in the art and, for instance, given in Sambrook *et al.* supra.

The immunotherapeutic agent of the present invention is suitable for the treatment of bladder carcinoma, e.g., non-invasive bladder carcinoma, e.g. non-invasive papillary carcinoma *in situ* (Tₐ), non-invasive carcinoma in situ (T_{cis}), tumor invading subepithelial connective tissue (T₁), tumor invading superficial muscle (inner half) (T₂ₐ), tumor invading deep muscle (outer half) (T_{2b}), tumor invading perivesical tissue (T₃ including T₃ₐ and T_{3b}), tumor invading prostate, uterus or vagina (T₄ₐ), and tumor invading pelvic wall or abdominal wall (T_{4b}). Particularly, the tumor is a superficial tumor or carcinoma in situ (T_{cis}), non-invasive papillary carcinoma (Tₐ), or a tumor invading subepithelial connective tissue (T₁). The immunotherapeutic treatment is suitable for the treatment of primary tumors and/or for the treatment of recurring tumors.

The immunotherapeutic agent is locally administered to the tumor site, i.e., to the site of a primary tumor before surgery or after surgery and optionally after chemotherapy. For the treatment of urothelial bladder carcinoma, the agent is preferably administered by vesicular instillation into the urinary bladder. For other tumors, the administration may involve local injection or, in case of lung tumors, pulmonal administration.

The immunotherapeutic agent of the invention may be administered as a first-line immunotherapy in patients, who have not been treated previously with an antitumor-immunotherapeutic agent such as standard BCG, or a follow-up immunotherapy in patients who have been treated previously with antitumor-immunotherapeutic agent such as standard BCG. The immunotherapeutic agent may be administered with a newly diagnosed bladder carcinoma, or to patients, particularly patients with recurrent bladder carcinoma.

The immunotherapeutic agent is administered to the subject to be treated in an effective dose. For a human subject, the dose for an administration may be about 10⁶ to 10¹⁰ viable units (CFU), e.g. about 10⁷ to 10⁹ or 10⁸ to 10⁹ viable units. Preferably, the immunotherapeutic agent is administered several times, e.g. at least 3 times or at least 5 times up to 30 times, particularly about 15 times, at predetermined times during the treatment.

The immunotherapeutic agent is usually provided as a pharmaceutical preparation which comprises the recombinant *M. bovis* BCG cell in solid form, e.g., a lyophilized or cryoconserved preparation, which is reconstituted with a suitable liquid carrier before use. Alternatively, the preparation may be provided in liquid form, e.g., as suspension.

In one embodiment, the immunotherapeutic agent of the invention is administered for the treatment of carcinoma *in situ.* A standard schedule may comprise weekly administration of the agent for at least 4, e.g., 4, 5, 6, 7 or 8 weeks as an induction therapy. The induction therapy should not start until 2-3 weeks after primary tumor surgery. After a treatment-free interval of, e.g., 4 weeks, administration may continue using maintenance therapy for at least 6 months or at least 1 year.

In a further embodiment, the immunotherapeutic agent is administered in an induction therapy in the prophylactic treatment of tumor recurrence. In this embodiment, therapy may start about 2-3 weeks after biopsy of the tumor site and be repeated, e.g., at weekly intervals for at least 4, e.g., 4, 5, 6, 7 or 8 weeks. In intermediate and high-risk tumors this may be followed by maintenance therapy.

Maintenance therapy may comprise long-term therapy, e.g., 6, 9 or 12 months therapy or even longer with treatments at monthly intervals. Alternatively, maintenance therapy may comprise 2, 3 or 4 administrations at weekly intervals, at month 3, 6, 12, 18, 24, 30 and 36.

In still a further embodiment, the immunotherapeutic agent, particularly recombinant BCG ΔUrec::Hly+, is used for the treatment of non-muscle invasive bladder cancer in patients with recurrence after standard BCG therapy. The immunotherapeutic agent is administered into the bladder according to a schedule involving weekly instillations during an induction phase with e.g. 6 weekly instillations, a first maintenance phase after about 3 months with e.g. 3 weekly instillations, a second maintenance phase after about 6 months with e.g. 3 instillations and a third maintenance phase after about 12 months with e.g. 3 instillations.

The administration as immunotherapeutic agent of the recombinant *M. bovis* BCG cell to site of a solid tumor as described above, may be combined with further antitumor therapy, e.g., radiation and/or chemotherapy. Further, the immunotherapy as described above, may be combined with a non-tumor site specific administration of the recombinant *M. bovis* BCG cell in order to provide a general stimulation of the immune system. This non-site specific administration may be effected as described in WO 2012/085101, e.g. before surgery of the primary tumor. In this case, the agent is preferably administered to a human subject in a dose of about 1-10 x 10⁵, preferably about 2-8 x 10⁵ cells. The agent is preferably administered as a single dose, e.g., by injection. Subcutaneous injection is preferred. Further it is preferred to administer the agent without adjuvant.

Further, the invention is described in more detail by the following Figures and Examples 1-3.

The immunotherapeutic agent *"rBCG"* used in these examples is recombinant *M*. *bovis* (BCG) Danish subtype Prague with an inactivated ureC sequence (ΔUrec) and without functional selection marker gene which expresses an Ag85B/Hly fusion protein as shown in SEQ ID No.2 (Hly+).

### Example 1 Single Dose Toxicity Study of recombinant BCG (rBCG) in Rats following Intravesicular Instillation

### 1.1 Conduct of study

| | | |
|---|---|---|
| Test item | rBCG lyophilized (rBCG Danish subtype Prague ΔUrec::Hly+ w/o functional selection marker gene) | |
| Approximate viable counts | 5.41×10⁸ CFU/vial | |
| Reference item | BCG medac | |
| Approximate viable counts | 2×10⁸ to 2×10⁹ CFU/vial | |
| Test species / Strain / Stock | Rat / CD® / Crl:CD(SD) | |
| Breeder Charles River Laboratories, Research Models, and Services, Germany GmbH Sandhofer Weg 7 97633 Sulzfeld, Germany | | |
| Number and sex of animals | 23 female animals; | |
| | 3 animals for group 1; | |
| | 5 animals for groups 2 to 5. | |
| Dose regime | Group 1: | Control (diluent) |
| | Group 2: | ∼2×10⁶ CFU rBCG (lyophilized)/animal |
| | Group 3: | ∼2×10⁸ CFU rBCG (lyophilized)/animal |
| | Group 4: | ∼2×10⁶ CFU rBCG (frozen w/o cryoprotectant)/ animal |
| | Group 5: | ∼2×10⁶ CFU BCG medac/animal |
| Route of administration | Intravesical instillation in the bladder | |
| Frequency of administration | Single dose on test day 1. | |
| Administration volume | 500 µL/animal | |
| Duration of study | • 12 adaptation days | |
| | • 4 in-life test weeks | |
| | • 28 incubation days | |

### 1.2 Results

| | |
|---|---|
| Mortality | None of the animals died prematurely. |
| Clinical signs | No changes of behaviour, external appearance or condition of faeces were observed for any animal at any treatment. |
| Body weight | The body weight of all animals of all dose groups was in the normal range throughout the course of the study. |
| Food and drinking water consumption | The food intake of all animals of all dose groups was in the normal range throughout the course of the study. |
| | The visual appraisal of the drinking water consumption did not reveal any test or reference item-related influence. |
| IL-2 levels | The IL-2 levels in urine and serum of all animals of all groups were below the lower limit of quantification. |
| Macroscopic *post mortem* findings | No test or reference item-related changes were noted. |
| Organ weights | No test or reference item-related changes were noted. |
| CFU counts | rBCG (groups 2 to 4) vs. control (group 1) |
| | No test-item related CFU counts were noted for the examined organs and the blood of the animals treated once with an intravesical instillation of **2×10⁶** or **2×10⁸ CFU rBCG (lyophilized)/animal,** or of **2×10⁶ CFU rBCG (frozen)/animal.** In particular, no CFU counts at all were noted for the urinary bladder four weeks after instillation of the test item, indicating a rapid clearance of the administered mycobacteria from the site of instillation. |
| | No differences were noted between the animals treated with **2x10⁶** or **2x10⁸CFU rBCG (lyophilized)/animal** or with **2x10⁶ CFU rBCG (frozen)/animal** and the control animals. |
| | BCG medac (group 5) vs. control (group 1) No reference-item related CFU counts were noted for the examined organs and the blood of the animals treated once with an intravesical instillation of **2×10⁶ BCG medac/animal.** In particular, no CFU counts at all were noted for the urinary bladder four weeks after instillation of the reference item, indicating a rapid clearance of the administered mycobacteria from the site of instillation. |
| | No differences were noted between the animals treated with **2x10⁶ BCG medac/animal** and the control animals. |
| | rBCG (groups 2 to 4) vs. BCG medac (group 5) No difference in CFU counts was noted for the examined organs and the blood of the animals treated once with an intravesical instillation of **2×10⁶** or **2×10⁸** CFU **rBCG (lyophilized) /animal,** or of **2×10⁶ CFU** rBCG **(frozen) /animal** compared to the reference group treated in the same way with **2×10⁶** CFU BCG **medac/animal.** |
| Immunohistochemistry | Immunohistochemistry for lymphocyte sub-typing in the urinary bladder tissue revealed the highest incidence of focal and multifocal lymphocytic infiltration with CD4 and CD8 positive cells in groups 2 and 3 treated once with **2×10⁶** or **2×10⁸** CFU rBCG **(lyophilized)/animal** by intravesical instillation, whereas animals of groups 1 (control), 4 and 5 expressed CD4 and CD8 positive lymphocytes only as single cell infiltrate levels (diffuse infiltration). Nearly all animals (12 of 13) of groups 1 (control), 4 and 5 expressed CD4 and CD8 positive lymphocytes only as single cell infiltrate levels (diffuse infiltration). In contrast nearly all animals (9 of 10) of groups 2 and 3 contained CD4 and CD8 positive lymphocytes in focal or multifocal lymphocyte infiltrates in addition to the diffuse infiltration. Neutrophilic granulocytes could rarely be detected in any slides examined. |

In conclusion, no signs of toxicity were noted for the animals treated with the test item, the reference item or the dilutent. There were no differences in the systemic spread of mycobacteria in blood and organs between **rBCG** and **BCG medac** for the treatment by intravesical instillation. No test-item related CFU counts were noted for the examined organs and the blood of the animals treated once with an intravesical instillation of **rBCG** compared to the control animals. In particular, no CFU counts at all were noted for the urinary bladder 4 weeks after instillation of the test item, indicating a rapid clearance of the administered mycobacteria from the site of instillation.

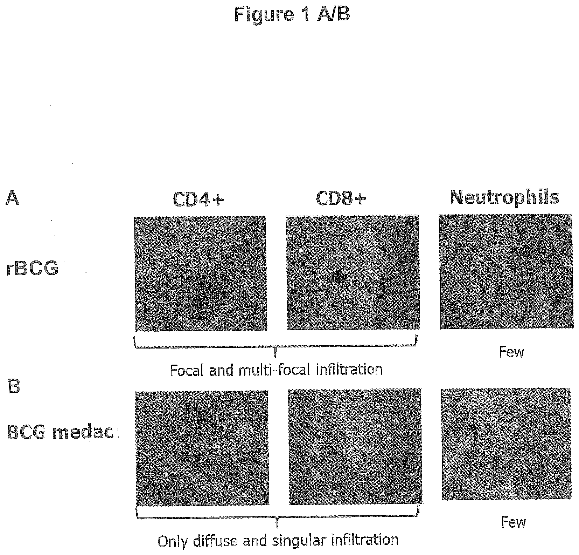

Immunohistochemistry for lymphocyte sub-typing in the urinary bladder tissue revealed the highest incidence of focal and multifocal lymphocytic infiltration with CD4 and CD8 positive cells in groups 2 and 3 treated once with **2×10⁶** or **2×10⁸ CFU rBCG/animal** by intravesical instillation, whereas animals of groups 1 (control), 4 and 5 expressed CD4 and CD8 positive lymphocytes only as single cell infiltrate levels (diffuse infiltration). Nearly all animals (12 of 13) of groups 1 (control), 4 and 5 expressed CD4 and CD8 positive lymphocytes only as single cell infiltrate levels (diffuse infiltration). In contrast, nearly all animals (9 of 10) of groups 2 and 3 contained CD4 and CD8 positive lymphocytes in focal or multifocal lymphocyte infiltrates in addition to the diffuse infiltration. Fig. 1A shows focal and multifocal lymphocytic infiltration after administration of rBCG. Fig. 1B shows only diffuse and singular infiltration after administration of BCG medac (200x magnification).

### Example 2 Repeated Dose Toxicity Study of recombinant BCG (rBCG) in Rats following Intravesicular Instillation

### 2.1 Conduct of study

| | |
|---|---|
| Test item | rBCG lyophilized |
| | (rBCG Danish subtype Prague ΔUrec::Hly+ w/o functional selection marker gene) |
| Approximate viable counts | 5.41×10⁸ CFU/vial |
| Reference item | BCG medac |
| Approximate viable counts | 2×10⁸ to 2×10⁹ CFU/vial |
| Test species / Strain / Stock | Rat / CD®/ Crl:CD(SD) |
| Breeder | Charles River Laboratories, Research Models, and Services, Germany GmbH Sandhofer Weg 7 97633 Sulzfeld, Germany |
| Number and sex of animals | 23 female animals; |
| | 3 animals for group 1; |
| | 5 animals for groups 2 to 5. |
| Dose regime | Group 1: Control (diluent) |
| | Group 2: ∼2×10⁶ CFU rBCG (lyophilized)/animal |
| | Group 3: ∼2×10⁸ CFU rBCG (lyophilized)/animal |
| | Group 4: ∼2×10⁶ CFU rBCG (frozen w/o cryoprotectant)/ animal |
| | Group 5: ∼2×10⁶ CFU BCG medac/ animal |
| Route of administration | Intravesical instillation in the bladder |
| Frequency of administration | Repeated administration; once weekly on test days 1, 8, 15, 22, 29, and 36. |
| Administration volume | 500 µL/animal |
| Duration of study | • 21 adaptation days |
| | • 9 in-life test weeks |
| | • 28 incubation days |

### 2.2 Results

| | |
|---|---|
| Mortality | None of the animals died prematurely. |
| Clinical signs | No changes of behaviour, external appearance or condition of faeces were observed for any animal at any treatment. |
| Body weight | The body weight of all animals of all dose groups was in the normal range throughout the course of the study. |
| Food and drinking water consumption | The food intake of all animals of all dose groups was in the normal range throughout the course of the study. |
| | The visual appraisal of the drinking water consumption did not reveal any test or reference item-related influence. |
| IL-2 levels | The IL-2 levels in urine and serum of all animals of all groups were below the lower limit of quantification. |
| Delayed type hypersensitivity (DTH assay) | No delayed type hypersensitivity was noted. |
| Macroscopic *post mortem* findings | No test or reference item-related changes were noted. |
| Organ weights | No test or reference item-related changes were noted. |
| CFU counts | rBCG (groups 2 to 4) vs. control (guroup 1) No test-item related CFU counts were noted for the examined organs and the blood of the animals treated six times with an intravesical instillation of **2×10⁶** or **2×10⁸** CFU rBCG (lyophilized)/animal, or of **2×10⁶** CFU rBCG **(frozen/w/o cryoprotectant)/animal.** In particular, no CFU counts at all were noted for the urinary bladder four weeks after the last instillation of the test item, indicating a rapid clearance of the administered mycobacteria from the site of instillation. No differences were noted between the animals treated with **2x10⁶** or **2x10⁸ CFU** |
| | **rBCG (lyophilized)/animal** or with **2x10⁶ CFU rBCG (frozen)/animal** and the control animals. |
| | BCG medac (group 5) vs. control (group 1) No reference-item related CFU counts were noted for the examined organs and the blood of the animals treated six times with an intravesical instillation of **2×10⁶ BCG medac/animal.** In particular, no CFU counts at all were noted for the urinary bladder four weeks after the last instillation of the reference item, indicating a rapid clearance of the administered mycobacteria from the site of instillation. No differences were noted between the animals treated with **2x10⁶ BCG medac/animal** and the control animals. |
| | rBCG (groups 2 to 4) vs. BCG medac (group 5) No difference in CFU counts was noted for the examined organs and the blood of the animals treated six times with an intravesical instillation of **2×10⁶** or **2×10⁸** CFU **rBCG (lyophilized)/animal,** or of **2×10⁶** CFU **rBCG (frozen)/animal** compared to the reference group treated in the same way with **2×10⁶ CFU BCG medac/animal.** |
| Immunohistochemistry | Immunohistochemistry for lymphocyte sub-typing in the urinary bladder tissue revealed the highest incidence of multifocal lymphocytic infiltration with CD4 and CD8 positive cells in group 2 treated with **2×10⁶ CFU rBCG/animal** by 6 intravesical instillations, followed by group 3 treated with **2×10⁸ CFU rBCG/animal** by 6 intravesical instillations, whereas animals of groups 1 (control), 4 and 5 expressed CD4 and CD8 positive lymphocytes only as single cell infiltrate levels (diffuse infiltration). Neutrophilic granulocytes could rarely be detected in any slides examined. |

In conclusion, no signs of toxicity were noted for the animals treated with the test item, the reference item or the diluent. There were no differences in the systemic spread of mycobacteria in blood and organs between **rBCG** and **BCG medac** for the treatment by intravesical instillation. No test-item related CFU counts were noted for the examined organs and the blood of the animals treated six times with an intravesical instillation of **rBCG** compared to the control animals. In particular, no CFU counts at all were noted for the urinary bladder 4 weeks after the last instillation of the test item, indicating a rapid clearance of the administered mycobacteria from the site of instillation.

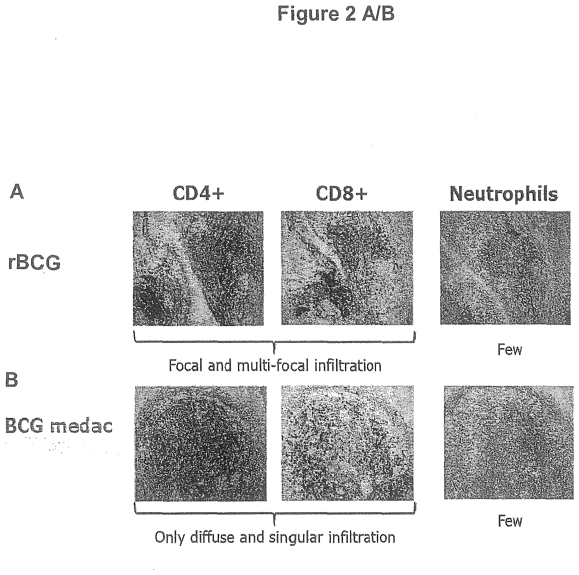

Immunohistochemistry for lymphocyte sub-typing in the urinary bladder tissue revealed the highest incidence of multifocal lymphocytic infiltration with CD4 and CD8 positive cells in group 2 treated with **2×10⁶ CFU rBCG/animal** by 6 intravesical instillations, followed by group 3 treated with **2×10⁸ CFU rBCG/animal** by 6 intravesical instillations, whereas animals of groups 1 (control), 4 and 5 expressed CD4 and CD8 positive lymphocytes only as single cell infiltrate levels (diffuse infiltration). Fig. 2A shows focal and multifocal lymphocytic infiltration after administration of rBCG. Fig. 2B shows only diffuse and singular infiltration after administration of BCG medac (200x magnification).

### Example 3 Phase I/II open label clinical trial assessing safety and efficacy of intravesical instillation of recombinant BCG (rBCG) in human patients with recurrent non-muscle invasive bladder cancer after standard BCG therapy

### 3.1 Clinical protocol

The recombinant BCG (as defined above) is currently applied in clinics as part of a Phase I/II clinical trial by instillation into bladder. The clinical trial phase I/II aims at assessing safety and efficacy of intravesical instillation of rBCG in human patients with recurrent non-muscle invasive bladder cancer after standard BCG therapy. rBCG is administered into bladder in 15 weekly instillations (induction phase: instillation 1-6, maintenance 3 months: instillation 7-9, maintenance 6 months: instillation 10-12, maintenance 12 months: instillation 13-15).

The primary endpoint of the phase I is dose limiting toxicity (DLT) of intravesical rBCG instillations in patients with recurrence after standard BCG therapy in non-muscle invasive bladder cancer. The DLT period corresponds to 3 instillations plus 1 week and covers acute toxicities induced by treatment. Patients are treated in two cohorts of three, following the rules of a 3 + 3 design (dose de-escalation rules: if patients treated at dose level 1 show signs of DLT, dose of instilled rBCG will be reduced to level -1, which is 10 times lower than level 1).

The dose levels are as follows:
- Dose level 1: 1 - 19.2 x 10⁸ CFUs of rBCG
- Dose level -1: 1 - 19.2 x 10⁷ CFUs of rBCG

### 3.2 Current status

The clinical trial currently is recruiting patients in trial sites in Switzerland (Basel, Geneva, Chur, Bern, Bellinzona, St. Gallen) within the frame of the Phase I.

The DLT period for the first three patients (cohort 1) was completed on 23 February 2016. Safety data, including DLT data, from this cohort has been collected by the sponsor and delivered to the Independent Safety Data Committee (ISDC). Since no DLT has been observed, the next three patients (second cohort) were allowed to be enrolled simultaneously and the recombinant BCG dose was maintained at dose level 1. An approval from the ISDC was received and all parties involved in the trial have been informed about this on 9 March 2016 by the sponsor. In addition, no tumor recurrence has been observed in this cohort of patients at the time of evaluation.

Currently recruitment of the second cohort is ongoing, with the first patient already enrolled in this cohort.

## Claims

1. A urease-deficient recombinant *Mycobacterium bovis* BCG cell from strain Danish subtype Prague which comprises a recombinant nucleic acid molecule encoding a fusion polypeptide comprising:
(a) a domain capable of eliciting an immune response comprising the amino acid sequence from aa.41 to aa.51 in SEQ ID No.2, and
(b) a Listeria phagolysosomal escape domain encoded by a nucleic acid molecule selected from
(i) a nucleotide sequence comprising nucleotides 211-1722 as shown in SEQ ID No.1,
(ii) a nucleotide sequence which encodes the same amino acid sequence as the sequence from (i), and
(iii) a nucleotide sequence hybridising under stringent conditions with the sequence from (i) or (ii)
for use in a human subject as immunotherapeutic agent in the treatment of bladder carcinoma, wherein the treatment comprises local administration of the immunotherapeutic agent by vesicular instillation into the urinary bladder, particularly after surgery.

2. The cell for use of claim 1, wherein the recombinant nucleic acid molecule does not comprise any functional selection marker.

3. The cell for use of claim 1 or 2, wherein the bladder carcinoma is non-invasive bladder carcinoma, particularly carcinoma *in situ* (T_{cis}), non-invasive papillary carcinoma (Tₐ), or a tumor invading subepithelial connective tissue (T₁).

4. The cell for use of any one of claims 1-3 for treating patients with newly diagnosed or recurrent bladder carcinoma who have not been treated previously with standard BCG.

5. The cell for use of any one of claims 1-4 for treating patients with recurrent bladder carcinoma who have been treated previously with standard BCG.

6. The cell for use of any one of claims 1-5, wherein the immunotherapeutic agent is administered into the bladder according to a schedule involving weekly instillations during (i) an induction phase with e.g. 6 weekly instillations, a first maintenance phase after about 3 months with e.g. 3 weekly instillations, a second maintenance phase after about 6 months with e.g. 3 instillations and a third maintenance phase after about 12 months with e.g. 3 instillations.

7. The cell for use of any one of claims 1-6, wherein the immunotherapeutic agent is used at a dose of from about 10⁶ to 10¹⁰ CFU per administration.

8. The cell for use of any one of claims 1-7, wherein the immunotherapy is combined with a non-tumor site specific administration of the recombinant *Mycobacterium bovis* cell.

9. The cell for use of any one of claims 1-8 for obtaining focal and/or multifocal lymphocytic infiltration at the site of administration, in particular for obtaining focal and/or multifocal tissue infiltration with CD4 and CD8 T cells.

## Patentansprüche

1. Urease-defiziente, rekombinante *Mycobacterium bovis* BCG Zelle vom Stamm Danish Subtyp Prague, die ein rekombinantes Nukleinsäuremolekül umfasst, das für ein Fusionspolypeptid kodiert, umfassend:
(a) eine Domäne, die in der Lage ist eine Immunantwort auszulösen, umfassend die Aminosäuresequenz von aa.41 bis aa.51 in SEQ ID Nr. 2 und
(b) eine phagolysosomale Listeria-Escapedomäne, die von einem Nukleinsäuremolekül kodiert ist, ausgewählt aus
(i) einer Nukleotidsequenz, umfassend die Nukleotide 211-1722, wie in SEQ ID Nr. 1 gezeigt,
(ii) einer Nukleotidsequenz, die für dieselbe Aminosäuresequenz wie die Sequenz aus (i) kodiert, und
(iii) einer Nukleotidsequenz, die unter stringenten Bedingungen mit der Sequenz aus (i) oder (ii) hybridisiert
zur Verwendung in einem humanen Subjekt als Immuntherapeutikum bei der Behandlung von Blasenkarzinom, wobei die Behandlung eine lokale Verabreichung des Immuntherapeutikums durch vesikuläre Instillation in die Harnblase, insbesondere nach einer Operation, umfasst.

2. Zelle zur Verwendung nach Anspruch 1, wobei das rekombinante Nukleinsäuremolekül keinen funktionellen Selektionsmarker umfasst.

3. Zelle zur Verwendung nach Anspruch 1 oder 2, wobei das Blasenkarzinom nichtinvasives Blasenkarzinom ist, insbesondere Carcinoma *in situ* (T_{cis}), nicht-invasives papilläres Karzinom (Tₐ) oder ein Tumor, der in subepitheliales Bindegewebe eindringt (T₁).

4. Zelle zur Verwendung nach einem der Ansprüche 1 bis 3 zur Behandlung von Patienten mit neu diagnostiziertem oder wiederkehrendem Blasenkarzinom, die zuvor nicht mit Standard-BCG behandelt worden sind.

5. Zelle zur Verwendung nach einem der Ansprüche 1 bis 4 zur Behandlung von Patienten mit wiederkehrendem Blasenkarzinom, die zuvor mit Standard-BCG behandelt worden sind.

6. Zelle zur Verwendung nach einem der Ansprüche 1 bis 5, wobei das Immuntherapeutikum in die Blase gemäß einem Zeitplan verabreicht wird, der wöchentliche Instillationen während (i) einer Induktionsphase mit z. B. 6 wöchentlichen Instillationen, eine erste Aufrechterhaltungsphase nach etwa 3 Monaten mit z. B. 3 wöchentlichen Instillationen, eine zweite Aufrechterhaltungsphase nach etwa 6 Monaten mit z. B. 3 Instillationen und eine dritte Aufrechterhaltungsphase nach etwa 12 Monaten mit z. B. 3 Instillationen umfasst.

7. Zelle zur Verwendung nach einem der Ansprüche 1 bis 6, wobei das Immuntherapeutikum in einer Dosis von etwa 10⁶ bis 10¹⁰ KBE pro Verabreichung verwendet wird

8. Zelle zur Verwendung nach einem der Ansprüche 1 bis 7, wobei die Immuntherapie mit einer nicht tumorstellenspezifischen Verabreichung der rekombinanten *Mycobacterium bovis* Zelle kombiniert ist.

9. Zelle zur Verwendung nach einem der Ansprüche 1 bis 8 zum Erhalten einer fokalen und/oder multifokalen lymphozytischen Infiltration an der Verabreichungsstelle, insbesondere zum Erhalten einer fokalen und/oder multifokalen Gewebeinfiltration mit CD4 und CD8 T-Zellen.

## Revendications

1. Cellule de BCG de *Mycobacterium bovis* recombinante déficiente en uréase de la souche Danish de sous-type Prague qui comprend une molécule d'acide nucléique recombinante codant un polypeptide de fusion comprenant:
(a) un domaine capable de déclencher une réponse immunitaire comprenant la séquence d'acides aminés de aa.41 à aa.51 dans SEO ID No. 2, et
(b) un domaine d'échappement phagolysosomal de Listeria codé par une molécule d'acide nucléique choisie parmi
(i) une séquence nucléotidique comprenant les nucléotides 211-1722 comme montré dans SEQ ID No. 1,
(ii) une séquence nucléotidique qui code la même séquence d'acides aminés que la séquence de (i), et
(iii) une séquence nucléotidique s'hybridant dans des conditions stringentes avec la séquence de (i) ou (ii)
destinée à être utilisée chez un sujet humain comme agent immunothérapeutique dans le traitement du carcinome de la vessie, où le traitement comprend l'administration locale de l'agent immunothérapeutique par instillation vésiculaire dans la vessie urinaire, en particulier après une chirurgie.

2. Cellule destinée à être utilisée selon la revendication 1, dans laquelle la molécule d'acide nucléique recombinante ne comprend aucun marqueur de sélection fonctionnel.

3. Cellule destinée à être utilisée selon la revendication 1 ou 2, dans laquelle le carcinome de la vessie est un carcinome de la vessie non invasif, en particulier un carcinome *in situ* (T_{cis}), un carcinome papillaire non invasif (Tₐ) ou une tumeur envahissant le tissu conjonctif sous-épithélial (T₁).

4. Cellule destinée à être utilisée selon l'une quelconque des revendications 1-3 pour traiter des patients ayant un carcinome de la vessie nouvellement diagnostiqué ou récurrent qui n'ont pas été traités auparavant avec du BCG standard.

5. Cellule destinée à être utilisée selon l'une quelconque des revendications 1-4 pour traiter des patients ayant un carcinome de la vessie récurrent qui ont été traités auparavant avec du BCG standard.

6. Cellule destinée à être utilisée selon l'une quelconque des revendications 1-5, dans laquelle l'agent immunothérapeutique est administré dans la vessie selon un programme impliquant des instillations hebdomadaires pendant (i) une phase d'induction avec par exemple 6 instillations hebdomadaires, une première phase d'entretien après environ 3 mois avec par exemple 3 instillations hebdomadaires, une seconde phase d'entretien après environ 6 mois avec par exemple 3 instillations et une troisième phase d'entretien après environ 12 mois avec par exemple 3 instillations.

7. Cellule destinée à être utilisée selon l'une quelconque des revendications 1-6, dans laquelle l'agent immunothérapeutique est utilisé à une dose d'environ 10⁶ à 10¹⁰ CFU par administration.

8. Cellule destinée à être utilisée selon l'une quelconque des revendications 1-7, dans laquelle l'immunothérapie est combinée avec une administration non spécifique de site tumoral de la cellule de *Mycobacterium bovis* recombinante.

9. Cellule destinée à être utilisée selon l'une quelconque des revendications 1-8 pour obtenir une infiltration lymphocytaire focale et/ou multifocale au site d'administration, en particulier pour obtenir une infiltration tissulaire focale et/ou multifocale avec des cellules T CD4 et CD8.


### SEQUENCE LISTING

<110> Vakzine Projekt Management GmbH
<120> Recombinant Mycobacterium as an immunotherapeutic agent for the
   treatment of cancer.
<130> 60182P EP
<160> 2
<170> BiSSAP 1.0
<210> 1
   <211> 1881
   <212> DNA
   <213> Artificial Sequence
<220>
   <221> source
   <222> 1..1881
   <223> /mol_type="DNA" /note="recombinant nucleic acid molecule" /organism="Artificial Sequence"
<220>
   <221> source
   <222> 1..1881
   <223> /mol_type="DNA" /note="CDS comprising a phagolysosomal domain (211-1722nt) and a stop codon (1879-1881)" /organism="Artificial Sequence"
<400> 1 
atgacagacg tgagccgaaa gattcgagct tggggacgcc gattgatgat cggcacggca 60

gcggctgtag tccttccggg cctggtgggg cttgccggcg gagcggcaac cgcgggcgcg 120

ttctcccggc cggggctgcc ggtcgagtac ctgcagtctg caaagcaatc cgctgcaaat 180

aaattgcact cagcaggaca aagcacgaaa gatgcatctg cattcaataa agaaaattca 240

atttcatcca tggcaccacc agcatctccg cctgcaagtc ctaagacgcc aatcgaaaag 300

aaacacgcgg atgaaatcga taagtatata caaggattgg attacaataa aaacaatgta 360

ttagtatacc acggagatgc agtgacaaat gtgccgccaa gaaaaggtta caaagatgga 420

aatgaatata ttgttgtgga gaaaaagaag aaatccatca atcaaaataa tgcagacatt 480

caagttgtga atgcaatttc gagcctaacc tatccaggtg ctctcgtaaa agcgaattcg 540

gaattagtag aaaatcaacc agatgttctc cctgtaaaac gtgattcatt aacactcagc 600

attgatttgc caggtatgac taatcaagac aataaaatcg ttgtaaaaaa tgccactaaa 660

tcaaacgtta acaacgcagt aaatacatta gtggaaagat ggaatgaaaa atatgctcaa 720

gcttatccaa atgtaagtgc aaaaattgat tatgatgacg aaatggctta cagtgaatca 780

caattaattg cgaaatttgg tacagcattt aaagctgtaa ataatagctt gaatgtaaac 840

ttcggcgcaa tcagtgaagg gaaaatgcaa gaagaagtca ttagttttaa acaaatttac 900
 
tataacgtga atgttaatga acctacaaga ccttccagat ttttcggcaa agctgttact 960

aaagagcagt tgcaagcgct tggagtgaat gcagaaaatc ctcctgcata tatctcaagt 1020

gtggcgtatg gccgtcaagt ttatttgaaa ttatcaacta attcccatag tactaaagta 1080

aaagctgctt ttgatgctgc cgtaagcgga aaatctgtct caggtgatgt agaactaaca 1140

aatatcatca aaaattcttc cttcaaagcc gtaatttacg gaggttccgc aaaagatgaa 1200

gttcaaatca tcgacggcaa cctcggagac ttacgcgata ttttgaaaaa aggcgctact 1260

tttaatcgag aaacaccagg agttcccatt gcttatacaa caaacttcct aaaagacaat 1320

gaattagctg ttattaaaaa caactcagaa tatattgaaa caacttcaaa agcttataca 1380

gatggaaaaa ttaacatcga tcactctgga ggatacgttg ctcaattcaa catttcttgg 1440

gatgaagtaa attatgatcc tgaaggtaac gaaattgttc aacataaaaa ctggagcgaa 1500

aacaataaaa gcaagctagc tcatttcaca tcgtccatct atttgccagg taacgcgaga 1560

aatattaatg tttacgctaa agaatgcact ggtttagctt gggaatggtg gagaacggta 1620

attgatgacc ggaacttacc acttgtgaaa aatagaaata tctccatctg gggcaccacg 1680

ctttatccga aatatagtaa taaagtagat aatccaatcg aatatgcatt agcctatgga 1740

agtcagggtg atcttaatcc attaattaat gaaatcagca aaatcatttc agctgcagtt 1800

ctttcctctt taacatcgaa gctacctgca gagttcgtta ggcgcggatc cggaattcga 1860

agcttatcga tgtcgacgta g 1881
<210> 2
   <211> 626
   <212> PRT
   <213> Artificial Sequence
<220>
   <221> SOURCE
   <222> 1..626
   <223> /mol_type="protein" /note="CORRESPONDING AMINO ACID SEQUENCE OF SEQ ID NO: 1." /organism="Artificial Sequence"
<400> 2

Met Thr Asp Val Ser Arg Lys Ile Arg Ala Trp Gly Arg Arg Leu Met
1               5                   10                  15
Ile Gly Thr Ala Ala Ala Val Val Leu Pro Gly Leu Val Gly Leu Ala
            20                  25                  30
Gly Gly Ala Ala Thr Ala Gly Ala Phe Ser Arg Pro Gly Leu Pro Val
        35                  40                  45
Glu Tyr Leu Gln Ser Ala Lys Gln Ser Ala Ala Asn Lys Leu His Ser
    50                  55                  60
Ala Gly Gln Ser Thr Lys Asp Ala Ser Ala Phe Asn Lys Glu Asn Ser
65                  70                  75                  80
Ile Ser Ser Met Ala Pro Pro Ala Ser Pro Pro Ala Ser Pro Lys Thr
                85                  90                  95
Pro Ile Glu Lys Lys His Ala Asp Glu Ile Asp Lys Tyr Ile Gln Gly
            100                 105                 110

Leu Asp Tyr Asn Lys Asn Asn Val Leu Val Tyr His Gly Asp Ala Val
        115                 120                 125
Thr Asn Val Pro Pro Arg Lys Gly Tyr Lys Asp Gly Asn Glu Tyr Ile
    130                 135                 140
Val Val Glu Lys Lys Lys Lys Ser Ile Asn Gln Asn Asn Ala Asp Ile
145                 150                 155                 160
Gln Val Val Asn Ala Ile Ser Ser Leu Thr Tyr Pro Gly Ala Leu Val
                165                 170                 175
Lys Ala Asn Ser Glu Leu Val Glu Asn Gln Pro Asp Val Leu Pro Val
            180                 185                 190
Lys Arg Asp Ser Leu Thr Leu Ser Ile Asp Leu Pro Gly Met Thr Asn
        195                 200                 205
Gln Asp Asn Lys Ile Val Val Lys Asn Ala Thr Lys Ser Asn Val Asn
    210                 215                 220
Asn Ala Val Asn Thr Leu Val Glu Arg Trp Asn Glu Lys Tyr Ala Gln
225                 230                 235                 240
Ala Tyr Pro Asn Val Ser Ala Lys Ile Asp Tyr Asp Asp Glu Met Ala
                245                 250                 255
Tyr Ser Glu Ser Gln Leu Ile Ala Lys Phe Gly Thr Ala Phe Lys Ala
            260                 265                 270
Val Asn Asn Ser Leu Asn Val Asn Phe Gly Ala Ile Ser Glu Gly Lys
        275                 280                 285
Met Gln Glu Glu Val Ile Ser Phe Lys Gln Ile Tyr Tyr Asn Val Asn
    290                 295                 300
Val Asn Glu Pro Thr Arg Pro Ser Arg Phe Phe Gly Lys Ala Val Thr
305                 310                 315                 320
Lys Glu Gln Leu Gln Ala Leu Gly Val Asn Ala Glu Asn Pro Pro Ala
                325                 330                 335
Tyr Ile Ser Ser Val Ala Tyr Gly Arg Gln Val Tyr Leu Lys Leu Ser
            340                 345                 350
Thr Asn Ser His Ser Thr Lys Val Lys Ala Ala Phe Asp Ala Ala Val
        355                 360                 365
Ser Gly Lys Ser Val Ser Gly Asp Val Glu Leu Thr Asn Ile Ile Lys
    370                 375                 380
Asn Ser Ser Phe Lys Ala Val Ile Tyr Gly Gly Ser Ala Lys Asp Glu
385                 390                 395                 400
Val Gln Ile Ile Asp Gly Asn Leu Gly Asp Leu Arg Asp Ile Leu Lys
                405                 410                 415
Lys Gly Ala Thr Phe Asn Arg Glu Thr Pro Gly Val Pro Ile Ala Tyr
            420                 425                 430
Thr Thr Asn Phe Leu Lys Asp Asn Glu Leu Ala Val Ile Lys Asn Asn
        435                 440                 445
Ser Glu Tyr Ile Glu Thr Thr Ser Lys Ala Tyr Thr Asp Gly Lys Ile
    450                 455                 460
Asn Ile Asp His Ser Gly Gly Tyr Val Ala Gln Phe Asn Ile Ser Trp
465                 470                 475                 480
Asp Glu Val Asn Tyr Asp Pro Glu Gly Asn Glu Ile Val Gln His Lys
                485                 490                 495
Asn Trp Ser Glu Asn Asn Lys Ser Lys Leu Ala His Phe Thr Ser Ser
            500                 505                 510
Ile Tyr Leu Pro Gly Asn Ala Arg Asn Ile Asn Val Tyr Ala Lys Glu
        515                 520                 525
Cys Thr Gly Leu Ala Trp Glu Trp Trp Arg Thr Val Ile Asp Asp Arg
    530                 535                 540
Asn Leu Pro Leu Val Lys Asn Arg Asn Ile Ser Ile Trp Gly Thr Thr
545                 550                 555                 560
Leu Tyr Pro Lys Tyr Ser Asn Lys Val Asp Asn Pro Ile Glu Tyr Ala
                565                 570                 575
Leu Ala Tyr Gly Ser Gln Gly Asp Leu Asn Pro Leu Ile Asn Glu Ile
            580                 585                 590
Ser Lys Ile Ile Ser Ala Ala Val Leu Ser Ser Leu Thr Ser Lys Leu
        595                 600                 605
Pro Ala Glu Phe Val Arg Arg Gly Ser Gly Ile Arg Ser Leu Ser Met

                610                 615                 620
Ser Thr
625